Europäisches Patentamt

European Patent Office

Office européen des brevets

(11) Publication number: **0 159 795**
A1

# EUROPEAN PATENT APPLICATION

(21) Application number: 85301797.8

(22) Date of filing: 14.03.85

(51) Int. Cl.⁴: **D 21 C 5/00**, C 12 P 19/14, C 13 K 13/00 // C12N9/24

(30) Priority: 16.03.84 GB 8406866

(43) Date of publication of application: 30.10.85 Bulletin 85/44

(84) Designated Contracting States: **AT BE CH DE FR GB IT LI NL SE**

(71) Applicant: **The British Petroleum Company p.l.c., Britannic House Moor Lane, London EC2Y 9BU (GB)**

(72) Inventor: **McCarthy, Alan John The University of, Manchester Institute of Science and Technology, P.O. Box 88 Manchester, M60 1QD (GB)**
Inventor: **Roberts, John Christopher The University of, Manchester Institute of Science and Technology, P.O. Box 88 Manchester, M60 1QD (GB)**

(74) Representative: **Ryan, Edward Terrence et al, c/o The British Petroleum Company plc Patents Division Chertsey Road, Sunbury-on-Thames Middlesex, TW16 7LN (GB)**

(54) Process for separating cellulose and hemicellulose.

(57) The hemicellulose content of a mixture of cellulose and hemicellulose is reduced by contacting the mixture with hemicellulase obtained by culturing the bacterium Saccharomonospora viridis in the absence of a cellulase-producing organism.

EP 0 159 795 A1

1

## PROCESS FOR SEPARATING CELLULOSE AND HEMICELLULOSE

The present invention relates to the removal of hemicellulose from mixtures with cellulose.

Cellulose is a valuable material which may be used as a raw material for the manufacture of artificial fibres and in the manufacture of paper. It is a polysaccharide built up from glucose units. It is sometimes found in nature in substantially pure form eg in cotton fibres. Often however it is found associated with hemicellulose.

The hemicelluloses most commonly encountered in nature are xylans.

Xylans are polysaccharides of the hemicellulose group and are a major component of the plant cell wall. They consist largely of beta-1,4 linked xylose units.

Some bacteria are known to attack and break down hemicelluloses, but such bacteria also usually attack cellulose. For some purposes this may be useful. However it would be desirable to find a way of breaking down hemicellulose selectively in the presence of cellulose. Purified cellulose is required in for example viscose rayon production. Wood pulps, used in paper production may contain up to 30% of hemicellulose eg xylan by weight, and if it were possible to reduce the hemicellulose content the properties of the paper might be significantly modified.

Removal of the hemicellulose without significantly affecting the cellulose fibre cannot be achieved by chemical treatment. It is known, for example, from Paice, M.G. _et al_, 1978, Applied and Environmental Microbiology 36, 802-808 to separate xylanase (a hemicellulose decomposing enzyme) from cellulolytic organisms such as cellulolytic fungi, but the separation and purification of cellulase-free xylanase is a difficult and time consuming operation.

We have now found a simple method of reducing the hemicellulose content of a mixture of cellulose and hemicellulose.

According to the present invention the process for reducing the hemicellulose content of a mixture of cellulose and hemicellulose comprises maintaining the mixture in contact with a solution of hemicellulase at a pH of 4-11 obtained by culturing the bacterium Saccharomonospora viridis in the absence of a cellulase-producing organism.

The mixture of cellulose and hemicellulose to which the present invention may be applied can be derived from a variety of plant materials, for example cereal straw, and timber pulps. Examples of timber pulps which may be used are larchwood and birchwood pulps.

The material subjected to the process of the present invention is preferably in finely divided form to give maximum contact between the hemicellulose and the hemicellulase.

The contact of the hemicellulose containing material with the hemicellulase solution may be carried out for example at temperatures in the range 10° to 85°C, preferably 50 to 70°C.

The contact time between the hemicellulose/cellulose mixture and the hemicellulase solution will depend on the specific material being treated and its state of subdivision. However, examples of suitable times are those in the range 5 to 48 hours, preferably 15 to 30 hours.

The pH of the mixture must be maintained at 4-11 for satisfactory activity, preferably 6.5-7.5, by incorporation of a suitable buffer.

The concentration of substances having an inhibiting effect on the hemicellulase is preferably kept low. In general reducing sugars inhibit the action of the hemicellulase. For this reason the initial hemicellulase solution brought into contact with the hemicellulose/cellulose mixture in preferably substantially free from reducing sugars. Reducing sugars will be produced as a result of the action of the hemicellulase on the hemicellulose. Preferably the process is operated so as to maintain the sugar content at a low level. Thus the solution may be subjected to a sugar removal step during the treatment of the hemicellulose/cellulose mixture. A sugar removal step is preferably applied to the solution of hemicellulase after it has been separated from the S. viridis cells and before it is brought into contact with the hemicellulose/cellulose mixture. Membrane separation processes (eg dialysis) may be used for sugar removal. The substantial absence of reducing sugar is shown by a negative result in the dinitrosalicylic acid colourimetric test.

The hemicellulase solution required by the process of the present invention may be obtained by culturing S. viridis in a suitable culture medium containing hemicellulose so that production and excretion of hemicellulase into the aqueous medium are induced. It is possible to produce hemicellulase by culturing the bacterium in media which do not contain hemicellulose but this is less effective. Cellular material can be removed by filtration or centrifugation. Alternatively, it is possible to culture S. viridis in liquid medium containing the hemicellulose/cellulose mixture so that production of hemicellulase and breakdown of hemicellulose occur almost simultaneously.

The micro-organism used in the process of the present invention is Saccharomonospora viridis. It is common in overheated substrates such as compost heaps, and can also be isolated from soil. The preferred strain is the type strain (P101, NCIB 9602) isolated by Kuester and Locci (1963). Archiv fuer Mikrobiologie 45, 188-197, and available from the National Collection of Industrial Bacteria at Torry Research Station, Aberdeen. Culture media and conditions suitable for culturing S. viridis will be well known to those skilled in the art and have been published. The culture medium on which the S. viridis is grown to produce the hemicellulase preferably contains hemicellulose as the main carbohydrate energy source. It is most desirable that the culture medium does not contain any substantial amounts of reducing sugars.

The process of the present invention may be used to regulate the hemicellulose content of wood pulps, eg, in the production of papers with reduced hemicellulose content and modified properties. It may be used as part of an analytical technique for the study of cellulosic materials by selective removal of hemicellulose. It may also be used where it is desired to exploit the hemicellulose content of materials by converting them into forms which can be more readily be degraded by other micro-organisms. Thus xyloses may be prepared from lignocellulosic wastes and then fermented to other products e.g. ethanol.

The invention will now be described by reference to the following examples.

Example 1

(a) Production of Xylanase (a hemicellulase)

Saccharomonospora viridis (NCIB 9602) was cultured on agar slopes of CYC medium (Cross & Attwell (1974) in Spore Research, 1973, pp 11-20) adjusted to pH 8.0. After incubation at 50°C for 72 hours, the growth was suspended in distilled water and used to inoculate a liquid medium which contained: $(NH_4)_2SO_4$, 2.0g;$MgCl_2$, $6H_2O$, 0.2g; CaCl, 11 mg; yeast extract, 1.0g; oatspelt xylan, 2.0g;Molar potassium phosphate buffer (pH 8.0), 100 ml;distilled water, 900 ml. Cultures were incubated at 50°C with shaking at 200 rpm for 72 hours. A supernatant liquid containing xylanase activity was harvested by shaking with glass beads for 30 minutes, followed by centrifugation at 7500 rpm for 20 minutes at 4°C. (Shaking with glass beads is designed to release any xylanase loosely adsorbed to the culture solids and gives approximately 20% increase in activity). The absence of cells in the supernatant liquid was confirmed by microscopic examination. Sodium azide was then added to a final concentration of 0.03% wt/vol, to inhibit microbial growth and thus preclude contamination. The supernatant liquid was then dialysed against a 0.2 molar sodium phosphate buffer to remove residual reducing sugar.

Dialysis is carried out to remove the products of growth on xylan (viz reducing sugars) which will inhibit xylanase activity. As growth time increases so sugars will be utilised by the growing organism. The need for dialysis depends on the sugar level in the culture supernatant liquid which in turn depends on the incubation time used to prepare the xylanase. Dialysis may not always be necessary if growth time is carefully adjusted and sugar levels monitored.

Xylanase activity and extracellular protein were measured by colourimetric procedures. Xylanase activity was determined by measuring, by well known techniques, the reducing sugars produced by xylan degradation. The tests were performed by incubating a portion of the cell-free supernatant liquid, phosphate buffer, (pH 7.0) and xylan at 50°-65°C for 15-30 minutes. Units of xylanase activity are expressed as micromoles of xylose equivalents produced per minute at 65°C and pH 7.0. Thin layer chromatography was also used to identify xylan degradation products.

Extracellular protein was determined by well known techniques.

Yields of xylanase were in the range 4-8 units/ml of supernatant liquid (70-300 units/mg of protein). Xylanase activity reached a peak after 72 hours and remained stable up to 160 hours incubation. When glucose or xylose was substituted for xylan in the culture medium, xylanase production was not induced.

(b) Removal of xylan from birchwood pulp

A fully bleached and beaten birchwood pulp, such as is used extensively in the paper industry, was found by high performance liquid chromatography to have a xylan content of approximately 20% weight, based on weight of solid matter in the pulp. The pulp was suspended in S. viridis culture supernatant liquid prepared as in (a) above, and diluted in a volume ratio of 1:1 with 0.2 molar sodium phosphate buffer (pH 7.0). The mixture was incubated at 50°C for 24 hours and monitored for the release of reducing sugar. Degradation of xylan was confirmed by thin-layer chromatography to detect xylose and xylan-derived oligomers. The pulp was washed in distilled water and subjected to a further three cycles of xylanase treatment resulting in the removal of 20% by weight of the total xylan originally present in the pulp. After the first cycle 12.5% wt of the xylan was removed, after the second cycle 4.4% was removed, after the third cycle 3.1% was removed and after the fourth cycle 0% by weight was removed.

Paper sheets were prepared from both treated and untreated pulps. Papers prepared from pulps whose xylan content has been reduced showed significant differences in properties from papers made from untreated pulps.

The long span tensile strength and burst factor decreased by 16% and 21% respectively. The zero span tensile strength was unaffected, indicating that the cellulose in the pulp had not been degraded. The air permeability and the scattering coefficient b were increased by 37% and 11.1% respectively. These measurements were made in accordance with British Standard methods used in the pulp and paper industry.

In addition to the above experiments, cotton pulp was treated with xylanase prepared by a method similar to that described above. The zero span tensile strength was tested as above. No reduction in zero span tensile strength was found as a result of the xylanase treatment. When the cotton pulp was treated with a commercial cellulase a large decrease in zero span tensile strength (compared with the tensile strength of untreated material) took place.

This is an indication that the enzyme produced in accordance with the invention does not have cellulase activity.

This was also indicated by sugar analysis by thin layer chromatography, on birch pulp treated in accordance with the invention, which showed the production of xylose but not glucose or cellobiose.

Claims:

1. The process for reducing the hemicellulose content of a mixture of cellulose and hemicellulose comprises maintaining the mixture in contact with a solution of hemicellulase at a pH of 4-11 obtained by culturing the bacterium <u>Saccharomonospora viridis</u> in the absence of a cellulase producing organism.

2. The process according to claim 1 wherein the mixture of cellulose and hemicellulose is timber pulp.

3. The process according to either one of claims 1 or 2 wherein the contact between the hemicellulose-containing material and the hemicellulase is carried out at a temperature in the range 10° to 85°C.

4. The process according to claim 3 wherein the temperature is in the range 50° to 70°C.

5. The process according to any one of the preceding claims wherein the contact time between the hemicellulose-containing material and the hemicellulose solution is in the range 5 to 48 hours.

6. The process according to any one of the preceding claims wherein the pH of the mixture of hemicellulose-containing mixture and hemicellulase solution is in the range 6.5 to 7.5

**European Patent Office**

**EUROPEAN SEARCH REPORT**

Application number

EP 85 30 1797

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 99, no. 16, 17th October 1983, page 99, no. 124357k, Columbus, Ohio, US; S. KAWATE et al.: "Enzymic hydrolysis of rice straw xylan" & TECHNOL. REP. KANSAI UNIV. 1983, 24, 199-207 | | D 21 C 5/00<br>C 12 P 19/14<br>C 13 K 13/00 //<br>C 12 N 9/24 |
| A | CHEMICAL ABSTRACTS, vol. 97, no. 19, 8th November 1982, page 551, no. 161028h, Columbus, Ohio, US; A.M. DESCHAMPS et al.: "Degradation of purified birch-wood xylan and production of xylanase by wood-decaying bacteria" & J. GEN. APPL. MICROBIOL. 1982, 28(3), 275-80 | | |
| P,X | CHEMICAL ABSTRACTS, vol. 101, no. 8, 20th August 1984, page 79, no. 56688b, Columbus, Ohio, US; M.G. PAICE et al.: "Removing hemicellulose from pulps by specific enzymic hydrolysis" & J. WOOD CHEM. TECHNOL. 1984, 4(2), 187-98 <br>* Abstract * | 1 | **TECHNICAL FIELDS SEARCHED (Int. Cl.4)**<br><br>D 21 C<br>C 12 P<br>C 13 K<br>C 08 B |

The present search report has been drawn up for all claims

| Place of search | Date of completion of the search | Examiner |
|---|---|---|
| THE HAGUE | 28-06-1985 | LENSEN H.W.M. |